# EUROPÄISCHE PATENTSCHRIFT

(11) **EP 1 531 331 B1**
(45) Veröffentlichungstag und Bekanntmachung des Hinweises auf die Patenterteilung: **02.01.2013**
(21) Anmeldenummer: 04025384.1
(22) Anmeldetag: 26.10.2004
(51) Int. Cl.: G01N 33/52, G01N 33/72, G01N 33/558

(54) **Verfahren zur Bestimmung von glycosyliertem Hämoglobin mittels einer Extraktionsschicht**
Method for the determination of glycosylated haemoglobin by means of an extraction layer
Procédé de détermination de l'hémoglobine glycosylée à l'aide d'une couche d'extraction

(30) Priorität: 31.10.2003 DE 10350880
(43) Veröffentlichungstag der Anmeldung: 18.05.2005
(73) Patentinhaber: Roche Diagnostics GmbH, 68305 Mannheim (DE); F. Hoffmann-La Roche AG, 4070 Basel (CH)
(72) Erfinder: Hoenes, Joachim, Dr., 64673 Zwingenberg (DE); Nortmeyer, Christine, 68305 Mannheim (DE); Horn, Carina, Dr., 68647 Biblis (DE)

(56) Entgegenhaltungen:
- EP-A- 0 455 225
- WO-A-2004/042364
- US-A- 4 042 335
- US-A- 4 255 384
- US-A- 4 522 786
- US-A- 4 587 102
- US-A- 4 806 311
- US-A- 4 895 704
- US-A- 5 059 525
- US-B1- 6 399 293

## Beschreibung

Die Anmeldung betrifft ein Verfahren zur Bestimmung von von glycosyliertem Hämoglobin als Analyten in Vollblut oder einen davon abgeleitetem Produkt als Flüssigkeitsprobe, welches insbesondere bei Vorliegen von weiteren, die Bestimmung des Analyten störenden Substanzen in der Flüssigkeitsprobe eine Analytbestimmung mit hoher Spezifität und Sensitivität ermöglicht. Weiterhin betrifft die Anmeldung ein Testsystem zur Bestimmung von Analyten in einer Flüssigkeitsprobe entsprechend den erfindungsgemäßen Verfahren sowie dessen erfindungsgemäße Verwendung.

### Stand der Technik

Der analytische Nachweis und die Konzentrationsbestimmung bestimmter biologisch und medizinisch relevanter Substanzen, sogenannter Analyten, aus komplexen Proben stellt eine wichtige Grundlage der modernen medizinischen Diagnostik dar.

Zur qualitativen und quantitativen Bestimmung von Analyten aus Flüssigkeitsproben, insbesondere aus Blut oder Urin, werden oft sogenannte trägergebundene Tests verwendet. Bei diesen trockenchemischen Verfahren liegen Reagenzien in trockener Form auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung oder Fluoreszenzänderung, welche visuell oder mit Hilfe eines Geräts, meist reflektionsphotometrisch oder fluorimetrisch, ausgewertet werden kann. Weitere Nachweismethoden beruhen auf elektrochemischen Methoden und erfassen beispielsweise Ladungs-, Potential- oder Stromänderungen. Solche Testträger sind häufig als Teststreifen ausgebildet, welche im wesentlichen aus einer länglichen Trägerschicht, einer die Nachweisreagenzien enthaltenen Nachweisschicht und evtl. weiteren Hilfsschichten, beispielsweise Filtrationsschichten, bestehen.

Ein Hauptproblem dieser analytischen Verfahren ist, dass der Analytnachweis in vielen Fällen nicht direkt in komplexen Probengemischen durchgeführt werden kann, da weitere neben dem Analyten in der Probenflüssigkeit vorhandene Substanzen die Analytbestimmung beeinflussen bzw. ganz verhindern können. In solchen Fällen ist in den bisherigen Verfahren eine vorausgehende Abtrennung des Analyten von diesen störenden Substanzen nötig, um überhaupt eine spezifische und sensitive Analyt bestimmung durchführen zu können. Solche der eigentlichen Analytbestimmung vorausgehende Aufreinigungsschritte erfordern oft zusätzliche Verfahrensschritte wie beispielsweise Waschschritte, Fällungsreaktionen, Zentrifugationsschritte, Adsorptionsschritte, Phasentrennungen oder Filtrationen, so dass solche Verfahren oft nur mit einem hohen zeitlichen, apparativen und personellen Aufwand durchzuführen sind. Daher ist es im Sinne einer möglichst einfachen und kostengünstigen Analytik wünschenswert, solche zusätzlich durchzuführenden Aufreinigungschritte zu vermeiden. Ein weiterer Nachteil durchgeführter Aufreinigungsschritte besteht darin, dass die Zusammensetzung der Probenflüssigkeit hierbei verändert wird. Dadurch kann insbesondere bei komplexen Nachweisreaktionen mit mehreren Reaktionsschritten und Reaktionspartnern, beispielsweise enzymatischen Reaktionskaskaden wie der Blutgerinnung, eine Analytbestimmung in der nativen Reaktionsumgebung nicht mehr gewährleistet werden, so dass eine Analytbestimmung verfälscht oder unmöglich wird.

In schichtweise aufgebauten Testsystemen werden häufig verschiedenste Blutparameter durch spezifische enzymatische Nachweisreaktionen und nachfolgende Detektion eines dabei gebildeten Produktes, der sogenannten Indikatorsubstanz, nachgewiesen. Voraussetzung hierfür aber ist, dass die Eigenschaften der Probeflüssigkeit, insbesondere das Vorhandensein anderer Substanzen in der gleichen Probe, die Detektion der Indikatorsubstanz nicht beeinflussen. So kann oft eine Analytbestimmung mittels optischer Verfahren in Blut nicht ohne komplexe Vorbehandlung der Probe durchgeführt werden, da das im Blut hochkonzentriert vorhandene Hämoglobin aufgrund seiner optischen Eigenschaften eine optische Bestimmung niedrig konzentrierter Analyten unmöglich machen. Daher ist in diesen Fällen bei den bisher angewandten Verfahren eine Abtrennung dieser Substanzen oder Zellen meist unumgänglich.

Ein beim Nachweis von Analyten, insbesondere mittels enzymatischer Reaktionen, häufig auftretendes Problem liegt darin, dass solche Reaktionsgemische oft sehr komplex zusammengesetzt sind, was einerseits zu einer geringen Stabilität und hohen Störanfälligkeit der Nachweisreaktion führt. Andererseits sind oft viele Substanzen, welche teilweise in äußerst geringen Konzentrationen im Reaktionsgemisch vorliegen oder deren Vorkommen oder Bedeutung sogar bisher noch unbekannt ist, nötig, um die Nachweisreaktionen in einer möglichst nativen Umgebung ablaufen zu lassen. Ein Beispiel hierfür ist die Gerinnungsreaktion. Für den Nachweis der im Laufe der Nachweisreaktionen umgesetzten Indikatorsubstanz in diesem Reaktionsgemisch ist diese komplexe Zusammensetzung wiederum nachteilig, da es zu verschiedensten Wechsel wirkungen der in der Probe beziehungsweise im Nachweisreagenzgemisch vorliegenden Substanzen kommen kann, welche wiederum die Indikatorsubstanzbestimmung stark beeinflussen können. Es müssen jeweils komplizierte Maßnahmen ergriffen werden, um solche störenden Bestandteile der Flüssigkeitsprobe abzutrennen, um zu verhindern, dass diese die Bestimmung der Indikatorsubstanz beeinflussen oder gar unmöglich machen. Insbesondere bei der Analytbestimmung in Vollblut bedeutet dies, dass eine zusätzliche Plasmaseparation somit oft unumgänglich ist.

Ein Beispiel hierfür ist die Blutzuckerbestimmung aus Vollblut mittels photometrischer Verfahren, welche auf dem Prinzip einer enzymatischen Umsetzung von Glucose, insbesondere durch Glucose-Oxidase oder Glucose-Dehydrogenase, im Rahmen einer farbbildenden Reaktion beruhen. Hierbei müssen die Blutzellen, insbesondere die roten Blutkörperchen, an der Oberfläche der Nachweisschicht abgefiltert werden, so dass nur das weitgehend farblose Plasma in die Nachweisschicht eindringt. Dort findet dann die farbgebende Reaktion statt, welche photometrisch gemessen werden kann. Hierzu müssen jedoch die roten Blutkörperchen sicher abgetrennt sein und deren rote Farbe durch zusätzliche Farbpigmente von der Nachweisschicht zurückgehalten werden.

Hierfür können beispielsweise Glasfaservliese, wie sie in EP 0045476 beschrieben sind, eingesetzt werden. Diese Glasfaservliese trennen störende Bestandteile, insbesondere Erythrozyten, bereits vor Beginn der Nachweisreaktionen ab. Das Funktionsprinzip beruht hierbei auf Filtrations- und Chromatographieeffekten.

Ein weiteres Beispiel ist die Bestimmung von Blutgerinnungsparametern, welche auf der Umsetzung eines fluoreszierenden Thrombinsubstrats beruht. Auch hier ist es nötig, störende Bestandteile der Probenflüssigkeit zu entfernen, insbesondere das stark absorbierende Hämoglobin. Solche Verfahren werden beispielsweise im Avocet-System zur Bestimmung von Gerinnungsparametern (Avocet Medical Inc., San Jose, CA, USA) eingesetzt. Hier werden die roten Blutkörperchen und das Hämoglobin durch eine Membran abgetrennt, während Plasma durch die Membran hindurchtreten kann und in den darunter liegenden Schichten die Nachweisreaktion zur Thrombinbestimmung stattfinden kann. In deren Verlauf wird dort ein fluoreszierendes Teilchen gebildet, welches den Verlauf der Blutgerinnung widerspiegelt. Die Messung kann nicht im Blut selbst durchgeführt werden, da Blut die Fluoreszenz vollständig unterdrücken würde. Auch hier erfolgt die Abtrennung der störenden Bestandteile aus der Probenflüssigkeit bereits vor Beginn der eigentlichen Nachweisreaktion.

EP 0974303 beschreibt ein Blutzuckermessinstrument zur Bestimmung des Blutzuckerspiegels auf Grundlage der Farbänderung einer Probe, wobei der Reagenzschichtabschnitt die eigentliche Reagenzschicht, die sich durch die Reaktion mit Blut verfärbt, und eine Filterschicht zum Filtern der Blutzellen des Blutes umfasst. Die Blutzuckerbestimmung erfolgt hier durch Auftragen eines Bluttropfens auf eine Auftragsöffnung, welcher anschließend die Reagenzschicht erreicht. Dort läuft die Nachweisreaktion ab, in deren Verlauf die Farbänderung der Probenflüssigkeit stattfindet. Anschließend wandert die Probenflüssigkeit weiter in die Filterschicht, in welcher Blutzellen heraufgefiltert werden. Der reflektionsphotometrische Nachweis der Farbänderung der Probe findet ebenfalls in der Filterschicht statt. EP 0974303 benutzt als Filterschicht insbesondere einen feinporigen Polyethersulfonfilm mit Porengrößen von 0,45 µm zur Abtrennung der Blutzellen. In diesem Fall erfolgt die Abtrennung unerwünschter Bestandteile durch rein physikalische Verfahren, d. h. unerwünschte Stoffe werden nach dem Prinzip des Größenanschlusses aus der Filterschicht herausgehalten. Bei derartig feinporigen Filtrationsverfahren ist es nachteilig, dass solche Filterschichten leicht zum Verschluss der Poren neigen. So kann es insbesondere bei hohen Konzentrationen von Partikeln in der Probe, wie es im Vollblut der Fall ist, zu einem raschen Verschluss der Filterschicht kommen, wodurch der Nachweis der Indikatorsubstanz stark beeinträchtigt wird, da diese mit zunehmendem Verschluss die Poren in immer geringerem Maße in die Nachweisschicht eindringen kann. Zu geringe Konzentrationswerte und mangelnde Reproduzierbarkeit, insbesondere in Abhängigkeit von der Ausgangsprobe, können somit die Folge solcher Verfahren sein.

Ramjee beschreibt in "The Use of Fluorogenic Substances to Monitor Thrombin Generation for the Analysis of Plasma and Whole Blood Coagulation" (Analytical Biochemistry 277, 11-18 (2000)) Methoden zur fluorimetrischen Bestimmung von Thrombin mittels fluoreszierender Thrombinsubstrate, welche direkt ohne Abtrennung störender Bestandteile der Probenflüssigkeit durchgeführt werden können. Als Probenflüssigkeiten können hierbei wiederaufgelöstes, lyophilisiertes, fibrinhaltiges Plasma, frisches Thrombozyten-abgereichertes Plasma, Thrombozyten-haltiges Plasma und Vollblut eingesetzt werden. Der Nachweis des Thrombins erfolgt durch Umsetzung eines schwach fluoreszierenden Thrombinsubstrats in ein stärker fluoreszierendes Reaktionsprodukt, welches als Indikatorsubtanz direkt in der Probenlösung nachgewiesen werden kann. Hierbei erfolgt keine Abtrennung störender Probenbestandteile, was bedeutet, dass die Nachweisreaktionen und die Bestimmung der Indikatorsubstanz beide im gleichen Kompartiment ablaufen, hier direkt in der Probenflüssigkeit. Der Thrombinnachweis ist hier nur als Flüssigtest und nicht auf trockenchemischer Basis beschrieben. Allerdings zeigt sich bei diesem Testsystem, dass hiermit eine Bestimmung von Thrombin im Vollblut nur sehr eingeschränkt möglich ist. Bei der Verwendung von Vollblut als Probenflüssigkeit wird im Vergleich zu Plasma die Fluoreszenz-Signalintensität auf weniger als ein Zehntel des Plasmawertes reduziert. Somit können mit diesen Verfahren keine analytischen Bestimmungen mit hoher Sensitivität im Vollblut durchgeführt werden. Diese starke Verminderung der gemessenen Fluoreszenzintensität im Vollblut ist durch eine Absorption des Anregungs- und Fluoreszenzlichtes durch chromogene Substanzen im Blut, insbesondere durch das im gleichen Kompartiment wie das Indikatorteilchen vorliegende Hämoglobin, hervorgerufen. Somit wäre zur Erhöhung der Sensitivität zusätzlich eine Abreicherung der störenden Substanzen, beispielsweise durch Filtration oder Zentrifugation, mit den zuvor beschriebenen Nachteilen nötig.

Die am 16.05.2002 beim Deutschen Patent- und Markenamt eingereichte Patentanmeldung mit dem Aktenzeichen DE 10221846.3 beschreibt ein Verfahren zum Nachweis eines Analyten in einer Probe durch eine enzymatische Reaktion mittels Inkontaktbringen der Probe mit einem Nachweisreagenz, welches ein Coenzym und ein katalytisch inaktives Coenzym-bindendes Protein enthält. Erfindungsgemäß wird das Coenzym durch Reaktion mit dem Analyten verändert und bindet in seiner veränderten Form an das katalytisch inaktive Coenzym-bindende Protein. Der Nachweis des veränderten Coenzyms wird zur Analytbestimmung herangezogen. Das katalytisch inaktive Coenzym-bindende Protein erfüllt hier zumindest teilweise die Funktion einer Fängersubstanz, indem sie an das veränderte Coenzym bindet, ohne es weiter umzusetzen. Die Fängersubstanz wird hier den übrigen Nachweisreagenzien zugesetzt. In einer speziellen Ausführungsform wird das gesamte Nachweisreagenz in eine Matrix eingelagert, so dass sich Fängersubstanz und restliche Nachweisreagenzien in einer gemeinsamen Schicht befinden.

US 4,042,335 oder auch US 4,255,384 beschreiben analytische Testelemente, welche jeweils eine Reagenzschicht zur Durchführung einer Nachweisreaktion und eine darunterliegende Nachweisschicht zur Detektion einer in der Reagenzschicht gebildeten Indikatorsubstanz aufweist. Diese Schichten werden durch eine separate radiationblocking layer getrennt, welche u.a. auch Blutzellen oder andere Substanzen von Eintritt in die Nachweisschicht abhält. Die Nachweisschicht kann weiterhin sogenannte "mordants" enthalten, welche die Indikatorsubstanz dort binden können. US 4,895,704 beschreibt einen ähnlichen Aufbau, bei welchen die Funktionen der separaten radiationblocking layer durch eine oder mehrere andere Schichten mitübernommen werden können.

US 4,806,311 beschreibt einen Aufbau zur Durchführung eines heterogenen Immunoassays, bei welchem der markierte Reaktionspartner in einer Detektionszone durch dort immobiliserte spezifische Bindepartner für diesen angereichert wird.

EP 0455225 beschreibt ein immunologisches Nachweisverfahren zur Bestimmung glycosylierter Proteine, bei welchen diese Analyten über einen spezifischen Antikörper an eine Festphase gebunden werden. Als spezifischer Marker für die festphasengebundenen glycosylierten Proteine werden in einen nachfolgenden Verfahrenschritt gelabelte Boronsäurederivate eingesetzt. Als Maß für die Menge/Anteil des glycosylierten Proteins wird die Menge der gelabelten Boronsäurederivate bestimmt, welches an den Analyten gebunden ist. Alternativ kann auch die nicht gebundene Fraktion der gelabelten Boronsäurederivate bestimmt werden.

### Aufgabe der Erfindung:

Die der vorliegenden Erfindung zugrunde liegende Aufgabe ist es, die geschilderten Nachteile des Standes der Technik wenigstens teilweise zu vermeiden. Insbesondere soll ein Verfahren zum Nachweis von von glykolsyliertem Hämoglobin als Analyten bereitgestellt werden, welches eine sensitive und spezifische Bestimmung dieses Analyten auch in komplexen Probenflüssigkeiten ermöglicht und den Bedürfnissen einer kostengünstigen und benutzerfreundlichen Routineanalytik gerecht wird.

### Erfindungsgemäße Lösung:

Diese Aufgabe wird erfindungsgemäß dadurch gelöst, dass den im Stand der Technik beschriebenen Bestandteilen eines Testsystems eine zusätzliche Extraktionsschicht hinzugefügt wird. Diese zusätzliche Extraktionsschicht ermöglicht es, die zur Analytbestimmung eingesetzte Indikatorsubstanz in den Bereichen des Testsystems selektiv anzureichern, in welchen der analytische Nachweis dieser Indikatorsubstanz erfolgt. Weiterhin ermöglicht die erfindungsgemäße Extraktionsschicht das Zurückhalten von Substanzen, welche eine Bestimmung der Indikatorsubstanz in der Nachweisschicht stören können. Insbesondere im Falle von im Blut vorkommenden Analyten können die Nachweisreaktionen zur Bestimmung des Analyten so tatsächlich in Vollblut stattfinden, während das dabei beteiligte Indikatorteilchen anschließend selektiv aus diesem Blutkompartiment in die Extraktionsschicht angereichert wird und dort möglichst störungsfrei nachgewiesen werden kann. Dadurch kann eine exakte und sensitive Bestimmung von Analyten auch ohne zusätzlich durchzuführende Abtrennungsschritte in einem einzigen Testsystem durchgeführt werden.

Ein Gegenstand der Erfindung ist somit ein Verfahren zur Bestimmung eines Analyten in einer Flüssigkeitsprobe mittels eines Testsystems bestehend aus mindestens zwei Kompartimenten, wobei in einem ersten Kompartiment die zur Bestimmung des Analyten notwendigen Nachweisreaktionen durchgeführt werden und in einem zweiten Kompartiment eine analytische Bestimmung einer an den Nachweisreaktionen beteiligten und vom Analyten verschiedenen Substanz, der Indikatorsubstanz, erfolgt, wobei die beiden Kompartimente in einer Weise voneinander abgegrenzt sind, welche den Übertritt der Indikatorsubstanz in das zweite Kompartiment ermöglicht und welche den Übertritt von weiteren Substanzen, welche die analytische Bestimmung der Indikatorsubstanz im zweiten Kompartiment stören können, zumindest teilweise verhindern und im zweiten Kompartiment mindestens eine weitere Substanz in immobilisierter Form vorliegt, welche als Fängersubstanz eine selektive Anreicherung der Indikatorsubstanz im zweiten Kompartiment bewirkt, wobei erfindungsgemäß der Analyt glycosyliertes Hämoglobin und die Flüssigkeitsprobe Vollblut oder ein davon abgeleitetes Produkt ist, im Laufe der Nachweisreaktionen im ersten Kompartiment eine niedermolekulare fluoreszenzmarkierte Boronsäure als Indikatorsubstanz vorliegt, welche spezifisch an glycosyliertes Hämoglobin bindet und die nicht an glycosyliertes Hämoglobin gebundene Indikatorsubstanz durch spezifische Fängersubstanzen, insbesondere Kohlenhydrate und Diole, im zweiten Kompartiment unter Ausschluss störender Probenbestandteile, insbesondere Blutzellen und chromophorer Substanzen wie Hämoglobin, und unter Ausschluß der an glycosyliertes Hämoglobin gebundene Indikatorsubstanz angereichert wird und der Nachweis der Indikatorsubstanz im zweiten Kompartiment mit optischen Methoden, insbesondere fluoreszenzoptischen Methoden, erfolgt.

Ein weiterer Gegenstand der vorliegenden Erfindung ist ein Testsystem zur Bestimmung von glycosyliertem Hämoglobin als Analyten in Vollblut oder einem davon abgeleitetem Produkt als Flüssigkeitsprobe, bestehend aus mindestens zwei Kompartimenten, wobei in einem ersten Kompartiment die zur Bestimmung von glycosyliertem Hämoglobin notwendigen Nachweisreaktionen durchgeführt werden und in einem zweiten Kompartiment eine analytische Bestimmung einer an den Nachweisreaktionen beteiligten und von glycosyliertem Hämoglobin verschiedenen Substanz, der Indikatorsubstanz, erfolgt, wobei die beiden Kompartimente in einer Weise voneinander abgegrenzt sind, welche den Übertritt der Indikatorsubstanz in das zweite Kompartiment ermöglicht und welche den Übertritt von weiteren Substanzen, welche die analytische Bestimmung der Indikatorsubstanz im zweiten Kompartiment stören können, zumindest teilweise verhindern und im zweiten Kompartiment mindestens eine weitere Substanz in immobilisierter Form vorliegt, welche als Fängersubstanz eine selektive Anreicherung der Indikatorsubstanz im zweiten Kompartiment bewirkt, wobei weiterhin erfindungsgemäß zur Durchführung der Nachweisreaktionen im ersten Kompartiment eine niedermolekulare fluoreszenzmarkierte Boronsäure als Indikatorsubstanz vorliegt, welche spezifisch an glycosyliertes Hämoglobin bindet und die nicht an glycosyliertes Hämoglobin gebundene Indikatorsubstanz durch spezifische Fängersubstanzen, insbesondere Kohlenhydrate und Diole, im zweiten Kompartiment unter Ausschluss störender Probenbestandteile, insbesondere Blutzellen und chromophorer Substanzen wie Hämoglobin, und unter Ausschluß der an glycosyliertes Hämoglobin gebundene Indikatorsubstanz angereichert wird und der Nachweis der Indikatorsubstanz im zweiten Kompartiment mit optischen Methoden, insbesondere fluoreszenzoptischen Methoden, erfolgt.

Das Funktionsprinzip des erfindungsgemäßen Testsystems lässt sich wie folgt darstellen:
Die Bestimmung des Analyten läuft nicht direkt ab, sondern wird durch eine Indikatorsubstanz vermittelt, welche im Verlauf analytspezifischer Nachweisreaktionen umgesetzt bzw. gebildet wird. Diese Nachweisreaktionen laufen nach Zusatz bzw. Freisetzung der nötigen Nachweisreagenzien, vorzugsweise ohne weitere Probenvorbereitung, direkt in der Probenflüssigkeit ab. Hierbei wird entweder direkt aus dem zu bestimmenden Analyten oder über eine komplexere Umsetzungskette, an welcher der Analyt beteiligt ist, eine Indikatorsubstanz in ihrer Konzentration verändert. Diese Indikatorsubstanz ist niedermolekular und befindet sich in einer Gleichgewichtsverteilung zwischen dem Reaktionsraum und der Extraktionsschicht, welche sich insbesondere mittels Diffusion einstellt. In der Extraktionsschicht immobilisierte Fängersubstanzen reichern selektiv die Indikatorsubstanz in der Extraktionsschicht an und erhöhen damit die Empfindlichkeit der Analytbestimmung erheblich. Somit können mit hoher Empfindlichkeit Analyten bestimmt werden, die selbst nicht in die Extraktionsschicht eindringen können: Andererseits werden sonstige Probenbestandteile, welche die Bestimmung der Indikatorsubstanz stören können, wie z.B. das stark absorbierende Hämoglobin, aus der Extraktionsschicht weitgehendst ausgeschlossen.

Testsysteme im Sinne der vorliegenden Anmeldung sind alle Vorrichtungen, welche eine Bestimmung eines Analyten in einer Flüssigkeitsprobe ermöglichen. Vorzugsweise sind solche Testsysteme trägergebunden aufgebaut. Solche Testträger sind häufig als Teststreifen ausgebildet, die im wesentlichen aus einer länglichen Tragschicht aus Kunststoffmaterial und darauf angebrachten Nachweiselementen als Testfeldern bestehen. Es sind jedoch auch Testträger bekannt, die als quadratische oder rechteckige Plättchen gestaltet sind. Eine besonders bevorzugte Ausführungsform besteht aus einem inerten Grundträger, auf welchen eine erfindungsgemäße Extraktionsschicht aufgebracht ist und welcher weiterhin einen Reaktionsraum aufweist, in welchem die Nachweisreaktionen unter Beteiligung des Analyten und der Indikatorsubstanz stattfinden und welcher somit die für die Nachweisreaktionen nötigen Substanzen enthält. Diese Reaktionsraum kann als spezielle Schicht des Testsystems vorliegen oder auch durch die Flüssigkeitsprobe selbst gebildet werden. In letzterem Fall können die Nachweisreagenzien in trockener Form auf dem Testträger, insbesondere auf oder innerhalb der Extraktionsschicht vorliegen und werden erst durch Aufbringen der Flüssigkeitsprobe gelöst und in den Reaktionsraum freigesetzt, womit die Nachweisreaktionen im Reaktionsraum in Gang gesetzt werden. Weiterhin können die benötigten Nachweisreagenzien in einem vorhergehenden Schritt der Flüssigkeitsprobe zugesetzt werden und anschließend wird dieses Reaktionsgemisch auf den Testträger, insbesondere auf die Extraktionsschicht aufgegeben.

Als erfindungsgemäßer Analyt kann glycosyliertes Hämoglobin bestimmt werden.

Flüssigkeiten im Sinne der vorliegenden Anmeldung können reine Flüssigkeiten und homogene oder heterogene Mischungen wie beispielsweise Dispersionen, Emulsionen oder Suspensionen sein. Insbesondere können in den Flüssigkeiten Atome, Ionen, Moleküle und Makromoleküle, insbesondere biologische Makromoleküle wie Nukleinsäuren, Peptide und Proteine, Lipide, Metabolite oder auch biologische Zellen oder Zellfragmente enthalten sein. Flüssigkeiten biologischer Herkunft sind Blut, Plasma, Serum, Urin, cerebrospinale Flüssigkeit, Tränenflüssigkeit, Zellensuspensionen, Zellüberstände, Zellextrakte, Gewebeaufschlüsse oder ähnliches. Flüssigkeiten können aber auch Kalibrationslösungen, Referenzlösungen, Reagenzlösungen oder Lösungen mit standardisierten Analytkonzentrationen, sogenannte Standards, sein.

Unter Bestimmung oder Nachweis von Substanzen versteht man in der vorliegenden Anmeldung sowohl einen qualitativen als auch einen quantitativen Nachweis von Substanzen. Insbesondere versteht man darunter eine Konzentrations- oder Mengenbestimmung der jeweiligen Substanz, wobei auch die Feststellung des Fehlens oder des Vorhandenseins einer Substanz als Bestimmung der Substanz angesehen wird.

Die Bestimmung von Analyten mittels spezifischer Nachweisreaktionen wird in der Analytik in bekannter Weise durchgeführt. Hierbei wird der zu bestimmende Analyt nicht direkt nachgewiesen, sondern der zu bestimmende Analyt ist an einer oder mehreren Nachweisreaktionen beteiligt, in deren Verlauf dieser eine vom Analyten verschiedene Indikatorsubstanz in einer Weise beteiligt ist, welche Rückschlüsse auf die Konzentration des Analyten ermöglichen.

Unter Nachweisreaktionen versteht man in der vorliegenden Anmeldung sowohl chemische als auch biochemische, insbesondere enzymatische, Reaktionen, an welchen der zu bestimmende Analyt beteiligt ist und in deren Verlauf die Konzentration einer vom Analyten verschiedenen Indikatorsubstanz in zur Konzentration des Analyten korrelierter Weise verändert wird. Die Indikatorsubstanz kann einerseits in einer einzigen Reaktion gebildet oder umgesetzt werden, an welcher der Analyt direkt beteiligt ist, andererseits kann die Indikatorsubstanz erst im Laufe einer Reaktionskaskade gebildet oder umgesetzt werden, in welcher der Analyt an einer anderen Stelle beteiligt ist. Die Korrelation der Menge der umgesetzten Indikatorsubstanz zur Menge des in der Probe enthaltenen Analyten erfolgt bevorzugt aufgrund stöchiometrischer Beziehungen. Eine Kalibrierung liefert einen Zusammenhang des Messwerts der Indikatorsubstanz mit der Konzentration des zu bestimmenden Analyten. Da die Konzentrationsänderung dieser Indikatorsubstanz in Korrelation zur Menge des Analyten steht, kann aus deren Bestimmung auf die Menge des vorhandenen Analyten geschlossen werden. Ein Beispiel für eine chemische Nachweisreaktion ist die Reaktion von glycosyliertem Hämoglobin (HbA_{1c}) mit einer fluoreszierenden Boronsäure, wodurch mit steigender HbA_{1c}-Konzentration in der Probe die Menge der freien Boronsäure in Korrelation zur HbA_{1c}-Menge abnimmt.

Neben der Konzentrationsbestimmung der Analyten selbst können mit den erfindungsgemäßen Verfahren auch die Geschwindigkeiten der Nachweisreaktionen, insbesondere die der Umsetzungen der Indikatorsubstanz, bestimmt werden. Aus diesen Werten können wiederum Aussagen über die Konzentrationen und Aktivitäten der beteiligten Substanzen, insbesondere beteiligter Enzyme, getroffen werden.

Als Indikatorsubstanz werden im Sinne der vorliegenden Anmeldung solche Substanzen bezeichnet, welche ein nachweisbares Signal bilden, welches mit der Menge oder Konzentration des Analyten korreliert. Als Indikatorsubstanzen können insbesondere chromogene oder fluoreszierende Substanzen eingesetzt werden. Chromogene Substanzen können insbesondere eine Farbe bilden, ihre Farbe verlieren oder ihre Farbe ändern. Fluoreszierende Substanzen können insbesondere eine Fluoreszenz ausbilden, ihre Fluoreszenz verlieren oder ihre fluoreszierenden Eigenschaften ändern. Insbesondere kann eine solche Indikatorsubstanz durch analytspezifische Reaktionen umgesetzt oder gebildet werden.

Die Nachweisreaktionen laufen erfindungsgemäß zumindest bis zur Bildung oder Umsetzung der Indikatorsubstanz in einem ersten Kompartiment, dem Reaktionsraum, ab. Der Reaktionsraum enthält neben der Flüssigkeitsprobe die zur Durchführung der Nachweisreaktionen, insbesondere die zur Bildung oder Umsetzung der Nachweissubstanz benötigten Substanzen und Hilfsmittel. Dieses Reagenzgemisch oder Teile dessen können in Form einer Lösung oder Suspension in einer wässrigen oder nichtwässrigen Flüssigkeit oder als Pulver oder Lyophilisat vorliegen. Weiterhin können sie in Form eines trockenchemischen Tests vorliegen, wobei das Reagenzgemisch auf einen Träger aufgebracht ist. Der Träger kann beispielsweise saugfähiges oder quellbares Material enthalten, welches von der Probenflüssigkeit benetzt wird, wodurch das sich in oder auf diesem Material in trockener Form befindliche Reagenzgemisch in der Probenflüssigkeit löst und die Nachweisreaktionen stattfinden können. Das Reagenzgemisch kann weiterhin in Form löslicher Schichten oder Filme auf ein Trägersubstrat aufgebracht werden. Die Nachweisreaktionen können durch das Inkontaktbringen von Probenflüssigkeit und Reagenzgemisch im Reaktionsraum direkt oder durch Zugabe einer für die Nachweisreaktionen essentiellen Substanz zu einem späteren Zeitpunkt gestartet werden. Der Gestaltung dieses Reaktionsraums unterliegt keinen Beschränkungen und kann von einem Fachmann auf dem Gebiet der apparativen Analytik dem jeweiligen Nachweisverfahren angepasst werden. So kann der Reaktionsraum beispielsweise als das Innere eines Reaktionsgefäßes, beispielsweise einer Küvette oder eines Mikrotiterplatten-Wells ausgebildet sein. Insbesondere bei einem trockenchemischen Teststreifen kann der Reaktionsraum auch dem Volumen der Flüssigkeitsprobe entsprechen. Wird beispielsweise ein Bluttropfen auf einen mit Nachweisreagenzien versehenen Teststreifen aufgebracht, löst dieser das Reagenzgemisch auf, wodurch die Nachweisreaktionen gestartet werden. In diesem Fall entspricht der Flüssigkeitstropfen selbst dem Reaktionsraum. Als Reaktionsraum ist somit unabhängig von seiner geometrischen Ausgestaltung das Volumen zu sehen, in welchem die Nachweisreaktionen zumindest bis zur Umsetzung oder Bildung der Indikatorsubstanz stattfinden.

Der Nachweis bzw. die Bestimmung der Indikatorsubstanz erfolgt erfindungsgemäß nicht im Reaktionsraum selbst, sondern in einem speziellen zweiten Kompartiment, der Extraktionsschicht. Diese Extraktionsschicht liegt räumlich getrennt vom Reaktionsraum vor, steht aber mit diesem in einer Weise in Kontakt, dass Flüssigkeiten sowie darin gelöste Stoffe zumindest unterhalb einer gewissen Ausschlussgröße vom Reaktionsraum in die Extraktionsschicht gelangen können. Die Extraktionsschicht erfüllt erfindungsgemäß mehrere Funktionen:
- Sie ermöglicht den Nachweis der Indikatorsubstanz in einer für diese Bestimmung besonders geeigneten Umgebung.
- Sie ermöglicht es, Substanzen, welche den Nachweis der Indikatorsubstanz stören können, am Übertritt in die Extraktionsschicht zu hindern, so dass der Nachweis der Indikatorsubstanz möglichst ungestört erfolgen kann.
- Sie enthält spezielle Substanzen, sogenannte Fängersubstanzen, welche eine selektive Anreicherung der Indikatorsubstanz in der Extraktionsschicht bewirken und somit die Sensitivität und Spezifität der Analytbestimmung erhöhen.

Die Extraktionsschicht hat somit unter anderem die Aufgabe, eine möglichst störungsfreie Bestimmung der Indikatorsubstanz zu ermöglichen. Hierzu ist es insbesondere nötig, einen räumlich abgegrenzten Bereich zu schaffen, in welchem einerseits die Indikatorsubstanz in einer Konzentration vorliegt, welche gut nachweisbar ist und zu ihrer Konzentration im Reaktionsraum korreliert und in welchem andererseits die Bestimmung störende Substanzen nicht oder nur in geringem Maße gelangen können, so dass eine Verfälschung der Bestimmung der Indikatorsubstanz durch diese störenden Substanzen weitgehendst vermieden wird.

Unter Substanzen, die eine Bestimmung der Indikatorsubstanz stören können, sind in Sinne der vorliegenden Anmeldung alle Substanzen oder Partikel zu verstehen, welche bei gemeinsamen Vorliegen zusammen mit der Indikatorsubstanz in der Flüssigkeitsprobe und/oder dem Reagenzgemisch eine Bestimmung der Indikatorsubstanz mit der jeweils angewanden Nachweismethode erschweren oder unmöglich machen. Solche Substanzen können beispielsweise mit der Indikatorsubstanz spezifisch oder unspezifisch wechselwirken, beispielsweise aufgrund hydrophober oder ionischer Wechselwirkungen, und hierdurch die im jeweiligen Nachweisverfahren genutzten Eigenschaften der Indikatorsubstanzen verändern. Ein Beispiel hierfür ist eine mögliche Veränderung der Fluoreszenzeigenschaften der Indikatorsubstanzen durch Wechselwirkung mit anderen Molekülen bzw. durch Veränderung des Mikromilieus der Indikatorsubstanzen durch solche störenden Substanzen, welche auf Prozessen wie Quenching oder Fluoreszenzresonanzenergietransfer bzw. lokalen Änderungen des pH-Wertes beruhen. Weitere solche störenden Wechselwirkungen können insbesondere auch mit in der Probe vorhandenen Proteinen auftreten und sind beispielsweise im "Handbook of Fluorescent Probes and Research Products" (9. Auflage, Molecular Probes Europe, Leiden, Niederlande) beschrieben. Störende Substanzen können jedoch auch Substanzen oder Partikel sein, welche aufgrund ihrer Eigenschaften eine Bestimmung der Indikatorsubstanz erschweren oder unmöglich machen, indem sie das Messsignal der Indikatorsubstanz zumindest teilweise überlagern und so das Messergebnis verfälschen. Insbesondere können im Reaktionsgemisch vorliegende chromogene Substanzen bei optischen Nachweisverfahren zusätzliche Messsignale hervorrufen, welche das Signal der Indikatorsubstanz überlagern, so dass nicht mehr zwischen dem Messsignal der Indikatorsubstanz und dem Einfluss störender Substanzen unterschieden werden kann, wodurch eine Bestimmung der Indikatorsubstanz erschwert oder unmöglich wird. Solche chromogenen Substanzen können bereits originär in der Probe vorliegen oder erst im Verlauf der Nachweisreaktionen gebildet werden. Beispielsweise sind Analytbestimmungen durch optische Nachweisverfahren in Vollblut nur sehr begrenzt durchführ- und auswertbar, da das im Vollblut in hohen Konzentrationen vorhandene Hämoglobin, über einen breiten Wellenlängenbereich stark absorbiert und so eine spezifische Absorption der Indikatorsubstanz, welche meist nur in geringen Mengen vorhanden ist, oft gänzlich überdeckt. Ähnliche Probleme ergeben sich auch bei Nachweisverfahren, welche auf fluoreszierenden Indikatorsubstanzen beruhen, da oft auch deren Anregungs- und Emissionslicht durch das hochkonzentrierte Hämoglobin nahezu vollständig absorbiert wird.

Die räumliche Trennung der Extraktionsschicht vom Reaktionsraum erfolgt erfindungsgemäß dadurch, dass die Extraktionsschicht als eine spezielle Schicht innerhalb des gesamten Testsystems ausgebildet ist, welche eine Diffusionsbarriere für bestimmte Substanzen darstellt, so dass der Übertritt von Substanzen vom Reaktionsraum in die Extraktionsschicht selektiv erfolgt. Unter selektiv ist im Sinne der vorliegenden Anmeldung nicht unbedingt ein vollständiger Ausschluss bestimmter Substanzen aus einer Schicht oder der vollständige Übertritt in eine Schicht zu verstehen. Selektiv ist auch in dem Sinne zu sehen, dass bestimmte Substanzen bevorzugt in die Schicht eindringen und sich dort anreichern können oder zumindest teilweise aus der Schicht abgehalten werden. Ein selektiver Ausschluss einer Substanz durch eine Schicht muss folglich nicht das vollständige Fehlen dieser Substanz in dem entsprechenden Kompartiment bedeuten, sondern lediglich eine verringerte Konzentration dieser Substanz im entsprechenden Bereich, da in der Praxis zumeist ein hundertprozentiger Ausschluss von Substanzen nicht erreicht werden kann beziehungsweise nicht benötigt wird.

Eine Abtrennung der Extraktionsschicht vom Reaktionsraum erfolgt insbesondere dadurch, dass die Extraktionsschicht eine Matrix mit einer selektiven Ausschlussgröße für Substanzen mit einem Molekulargewicht von mehr als 1.500 g/mol, vorzugsweise mehr als 2.000 g/mol, besonders vorzugsweise mehr als 15.000 g/mol, ist. Als solche Matrixmaterialien kommen insbesondere quell- und saugfähige Materialien in Frage, welche Flüssigkeit aufnehmen können. Dies können beispielsweise entsprechend feinporige fasrige Materialen wie Vliese, Gewebe, Gewirke oder poröse Kunststoffmaterialien sein. Insbesondere können solche Matrizen aber auch nicht faserige Materialien, wie poröse oder nicht-poröse Filme, Membranen, Gelmatrizen oder Polymerschichten sein. In einer besonders bevorzugten Ausführungsform ist die Extraktionsschicht als eine Gelmatrix ausgeführt, in welcher die weiteren Bestandteile der Extraktionsschicht, insbesondere die Fängersubstanzen, eingelagert sind. Die Gelmatrix weist vorzugsweise eine Schichtdicke von weniger als 50 µm, insbesondere von weniger als 5 µm, auf und ist auf einem Träger, beispielsweise einem zumindest teilweise optisch transparenten Träger aufgebracht. Die Gelmatrix ist vorzugsweise ein Polymer, das auf Basis von photopolymerisierbaren Monomeren, wie etwa acrylischen Monomeren, z. B. Acrylamid oder/und Acrylsäureestern, z. B. Polyethylenglykoldiacrylat, oder vinylaromatischen Monomeren, z. B. 4-Vinylbenzosulfonsäure, oder Kombinationen davon aufgebaut ist. Zur Herstellung einer derartigen Gelmatrix kann in einer bevorzugten Ausführungsform eine Flüssigkeit, welche ein oder mehrere photopolymerisierbare Monomere, Fängersubstanzen sowie gegebenenfalls weitere zusätzliche Bestandteile der Extraktionsschicht wie beispielsweise Farbpigmente enthält, auf einen zumindest teilweise optisch transparenten Träger, beispielsweise auf eine Plastikfolie, aufgetragen und z. B. mit UV-Licht von der Rückseite her bestrahlt werden, so dass eine Polymerisation der Monomere auf dem Träger bis zu einer vorbestimmten Schichtdicke erfolgt. Die Schichtdicke kann durch Zugabe von absorbierenden Substanzen zum Reagenz oder/und durch die Bestrahlungsdauer bzw. -intensität gesteuert werden. Überschüssiges flüssiges Reagenz kann nach der Polymerisation entfernt und erneut eingesetzt werden.

Andererseits kann die Matrix auch durch konventionelle Beschichtungsprozeduren hergestellt werden, wobei eine Suspension aller für die Extraktionsschicht benötigten Reagenzien auf einen Träger aufgetragen, dort mit geeigneten Methoden, z. B. mit einem Rakel, auf die gewünschte Dicke gebracht und dann getrocknet oder vollständig polymerisiert wird.

Neben kovalent vernetzten polymeren Gelmatrizen können auch nicht kovalent vernetzte Gele als Bestandteil der Extraktionsschichten herangezogen werden, beispielsweise Polyelektrolytgele, wie z.B. mit zweiwertigen Ionen vernetzte Alginatgele.

Weiterhin können als Matrixmaterialien Polyamid-, Polyvinylidendifluorid-, Polyethersulfon- oder Polysulfonmembranen eingesetzt werden. Die weiteren in der Extraktionsschicht enthaltenen Substanzen, insbesondere die Fängersubstanzen, können beispielsweise durch Imprägnierung in die Membranen eingebracht worden. Weiterhin können für die Extraktionsschicht sogenannte offene Filme eingesetzt werden, wie sie beispielsweise in EP-B-0 016 387 beschrieben sind. Hierfür werden einer wässrigen Dispersion von filmbildenden organischen Kunststoffen Feststoffe als feine, unlösliche, organische oder anorganische Partikel als Füllstoffe zugegeben und die weiteren in der Extraktionsschicht enthaltenen Substanzen, insbesondere die Fängersubstanzen, zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wässrigen Dispersionen. Die Dispersionen lassen sich auf einer Unterlage zu einer gleichmäßigen Schicht verstreichen, die nach dem Trocknen einen wasserfesten Film ergibt. Obwohl die weiteren in der Extraktionsschicht enthaltenen Substanzen, insbesondere die Fängersubstanzen, normalerweise in die zur Herstellung der offenen Filme verwendete Dispersion gegeben werden, kann es auch vorteilhaft sein, wenn der gebildete Film nach seiner Herstellung mit diesen Reagenzien imprägniert wird. Auch eine Vorimprägnierung der Füllstoffe mit den Reagenzien ist möglich.

Die Ausschlussgröße des Matrixmaterials kann in den vorigen Fällen beispielsweise durch die Wahl geeigneter Füllstoffe und deren Konzentration, durch Steuerung des Vernetzungsgrades von polymeren Gelmatrizen oder durch geeignete Konzentration von vernetzenden Ionen bei Polyelektrolytgelen in dem Fachmann bekannter Weise eingestellt und insbesondere den auszuschließenden störenden Substanzen angepasst werden.

Die Bestimmung der Indikatorsubstanz in der Extraktionsschicht kann mit verschiedensten, dem Fachmann auf dem Gebiet der apparativen Analytik bekannten optischen Nachweismethoden durchgeführt werden. Optische Methoden umfassen beispielsweise die Messung von Absorption, Transmission, Circulardichroismus, optische Rotationsdispersion, Refraktrometrie oder bevorzugt Fluoreszenz. Besondere Vorteile ergeben sich durch die erfindungsgemäßen Testsysteme und Verfahren für optische Methoden, welche unter der Lichtabsorption von störenden Substanzen, insbesondere Blutbestandteilen, besonders leiden.

Ein essentieller Bestandteil der Extraktionsschicht sind immobilisierte Fängersubstanzen. Unter Fängersubstanzen sind im Sinne der vorliegenden Anmeldung alle Substanzen zu verstehen, welche mit der Indikatorsubstanz wechselwirken und eine selektive Anreicherung dieser Substanz in der Extraktionsschicht bewirken können. Unter einer selektiven Anreicherung ist eine Anreicherung der Indikatorsubstanz zu verstehen, welche zu einer Konzentration dieser Substanz in der Extraktionsschicht führt, welche größer als die Konzentration ist, welche sich durch den rein diffusiven Einstrom dieser Substanz in die Extraktionsschicht einstellen würde. Insbesondere soll die Fängersubstanz die Indikatorsubstanz in dem Sinne selektiv anreichern, dass deren relative Konzentration in Bezug zu den anderen Bestandteilen der Probe bzw. des Reagenzgemisches in der Extraktionsschicht erhöht ist. Hierbei müssen die Fängersubstanzen nicht unbedingt substanzspezifisch mit der Indikatorsubstanz wechselwirken, wie dies beispielsweise bei Antigen-Antikörper-Wechselwirkungen der Fall ist. Die Spezifität kann auch Gruppen von Substanzen, welche gemeinsame Eigenschaften, insbesondere auf Grund einer ähnlichen chemischen Struktur oder ähnlicher physikochemischen Eigenschaften, besitzen, umfassen. Beispielsweise können durch kationische Fängersubstanzen prinzipiell alle anionischen Indikatorsubstanzen angereichert werden und umgekehrt durch anionische Fängersubstanzen alle kationischen Indikatorsubstanzen. Fängersubstanzen können somit auf Basis einer allgemeinen Wechselwirkung auch eine begrenzte Spezifität für die Indikatorsubstanz aufweisen.

Insbesondere können als Fängersubstanzen Substanzen eingesetzt werden, welche aufgrund von hydrophilen oder hydrophoben Wechselwirkungen spezifisch Indikatorsubstanzen anreichern können. Beispielsweise können Cyclodextrine oder Zuckerderivate als Fängersubstanzen eingesetzt werden, um aufgrund hydrophober Effekte selektiv Kohlenhydrate oder relativ hydrophobe Indikatorsubstanzen wie Aminocoumarine, Nitroaniline oder Phenylendiamine in der Extraktionsschicht anzureichern. Auch Serumalbumine können eingesetzt werden.

Weiterhin können als Fängersubstanzen Substanzen eingesetzt werden, welche aufgrund von ionischen Wechselwirkungen spezifisch Indikatorsubstanzen anreichern können. Beispielsweise können Polyelektrolyte wie Polykationen oder Polysulfonsäuren als Fängersubstanzen eingesetzt werden, um aufgrund elektrostatischer Wechselwirkungen selektiv entgegengesetzt geladene Indikatorsubstanzen in der Extraktionsschicht anzureichern.

Weiterhin können als Fängersubstanzen Substanzen eingesetzt werden, welche aufgrund komplexbildender Eigenschaften spezifisch Indikatorsubstanzen anreichern können. Beispielsweise können Chelatoren wie Ethylendiamintetraessigsäure-Derivate als Fängersubstanzen eingesetzt werden, um mehrwertige Ionen selektiv in der Extraktionsschicht anzureichern.

Weiterhin können als Fängersubstanzen Substanzen eingesetzt werden, welche aufgrund einer Fällungsreaktion spezifisch Indikatorsubstanzen anreichern können. Beispielsweise können Anionen oder Kationen spezifisch als Fängersubstanzen für Proteine eingesetzt werden, indem durch Zugabe geeigneter Salze ein gelöstes Protein ganz oder teilweise als Niederschlag in der Extraktionsschicht abgeschieden wird.

Weiterhin können als Fängersubstanzen Substanzen eingesetzt werden, welche aufgrund einer spezifischen Wechselwirkung zwischen den Partnern eines spezifischen Bindepaares nach dem Schlüssel-Schloss-Prinzip Indikatorsubstanzen anreichern können. Beispielsweise können Antikörper oder Antikörperfragmente spezifisch als Fängersubstanzen für spezielle Antigene oder Haptene eingesetzt werden. Weiterhin können Proteine, insbesondere Enzyme spezifisch als Fängersubstanzen für entsprechende Cofaktoren, insbesondere Coenzyme oder auch in inaktivierter Form für Substrate dieser Enzyme eingesetzt werden. Weiterhin können Nukleinsäuren, insbesondere DNA oder RNA spezifisch als Fängersubstanzen für entsprechende mit diesen Nukleinsäuren hybridisierende Nukleinsäuren, insbesondere komplementäre Nukleinsäuren, eingesetzt werden. Auch einzelne Partner anderer biologischer oder chemischer Bindepaare wie beispielsweise Biotin/Streptavidin oder Biotin/Avidin können als Fängersubstanzen eingesetzt werden.

Die Fängersubstanzen müssen erfindungsgemäß in immobilisierter Form in der Extraktionsschicht vorliegen. Unter Immobilisierung versteht man im Sinne der vorliegenden Anmeldung alle Prozesse und Vorkehrungen, die bewirken, dass die Fängersubstanzen in der Extraktionsschicht zurückgehalten werden und nicht in den Reaktionsraum gelangen können. Dies ist eine Grundlage für eine selektive Anreicherung der Indikatorsubstanzen in der Extraktionsschicht. Die Immobilisierung kann hierbei auf unterschiedliche Weise durchgeführt werden. Verfahren hierzu sind dem Fachmann bekannt. Insbesondere ist das Fixieren der Fängersubstanzen in der Extraktionsschicht eingeschlossen. Insbesondere kann die Immobilisierung durch Bindung an eine Trägersubstanz, vorzugsweise die Matrix der Extraktionsschicht, erfolgen. Die Bindung an einen Träger kann durch vorzugsweise durch Adsorption, Ionenbindung oder kovalente Bindung erfolgen. Weiterhin kann die Immobilisierung durch Einschluss in eine für die Fängersubstanzen nicht passierbare Schicht in Form von Gelen, Mikrokapseln oder Fasern erfolgen. Vorzugsweise ist diese immobilisierende Schicht die Matrix der Extraktionsschicht. Weiterhin kann die Immobilisierung durch Quervernetzung der Fängersubstanzen innerhalb der Extraktionsschicht erreicht werden.

Die Extraktionsschicht kann neben den Fängersubstanzen weitere Substanzen enthalten, welche die Nachweisbarkeit der Indikatorsubstanz in dieser Schicht erhöhen können. Beispielsweise können der Extraktionsschicht Farbpigmente zusetzt werden, um eventuelle störende Einflüsse dahinter liegender farbiger Schichten zu minimieren. Insbesondere kann dies bei optischen Nachweisverfahren zur Analytbestimmung in Vollblut vorteilhaft sein, um die starke Hintergrundfärbung durch das Hämoglobin zu minimieren. Hierzu können der Extraktionsschicht Pigmente mit einem hohen Brechungsindex zugesetzt werden. Vorzugsweise kann Titandioxid eingesetzt, wobei sich Teilchen mit einem mittleren Durchmesser von etwa 0,2 bis 0,8 µm als besonders vorteilhaft erwiesen haben.

Die Konzentration der Indikatorsubstanz in der Extraktionsschicht korreliert mit der Konzentration der Indikatorsubstanz im Reaktionsraum. Diese Konzentration der Indikatorsubstanz im Reaktionsraum korreliert wiederum mit der Konzentration des Analyten im Reaktionsraum, aus welcher wiederum bei bekannten Volumenverhältnissen von Probenflüssigkeit und Reaktionsgemisch auf die Konzentration des Analyten in der Flüssigkeitsprobe geschlossen werden kann. Somit ermöglicht eine Bestimmung der Indikatorsubstanz in der Extraktionsschicht die Berechnung der Konzentration des Analyten in der Flüssigkeitsprobe.

Die Bestimmung der Indikatorsubstanz und damit die Bestimmung des Analyten kann hierbei insbesondere durch eine einmalige Ermittlung eines Messwertes, insbesondere eine Endpunktsbestimmung oder eine Messung nach Ablauf eines bestimmten Zeitintervalls, oder durch eine Messung über einen bestimmten Zeitraum hinweg mit mehrmaliger Ermittlung von diskreten Messwerten in diskreten Zeitabständen oder durch eine Messung über einen bestimmten Zeitraum hinweg mit kontinuierlicher Ermittlung von Messwerten erfolgen.

An die Bestimmung des Analyten bzw. der Indikatorsubstanz können sich weitere Berechnungen anschließen, welche ausgehend von dem Ergebnis der Analytbestimmung weitere davon abgeleitete Größen liefern, welche ihrerseits als diagnostische Parameter genutzt werden können.

Neben diesen Verfahren ist ein Testsystem zur Bestimmung von Analyten in einer Flüssigkeitsprobe, welches aus mindestens zwei räumlich getrennten Kompartimenten besteht, wobei in einem ersten Kompartiment, dem Reaktionsraum, die zur Bestimmung des Analyten in der Flüssigkeitsprobe notwendigen Nachweisreaktionen, insbesondere unter Beteiligung der Indikatorsubstanz, durchgeführt werden und in einem zweiten Kompartiment, der Extraktionsschicht, die analytische Bestimmung der Indikatorsubstanz erfolgt, ein weiterer Gegenstand der vorliegenden Erfindung. Zur Erhöhung von Spezifität und Sensitivität enthält die Extraktionsschicht erfindungsgemäß zusätzlich eine Fängersubstanz in immobilisierter Form, welche eine selektive Anreicherung der Indikatorsubstanz bewirkt. Diese beiden Kompartimente sind erfindungsgemäß derartig räumlich getrennt, dass insbesondere der Übertritt der Indikatorsubstanz in Extraktionsschicht begünstigt ist, andererseits aber Substanzen, welche dort eine analytische Bestimmung der Indikatorsubstanz stören können, zumindest teilweise aus dieser ausgeschlossen bleiben.

Die oben genannte Aspekte der Erfindung können entweder alleine oder in beliebiger Kombination verwendet werden.

Die vorliegende Erfindung wird im nachfolgenden Beispiel näher erläutert.

### Beispiel 1: Bestimmung von glycosyliertem Hämoglobin(HbA_{1c}) mittels

HbA_{1c} ist ein Hämoglobin, an welches kovalent Glukose gebunden ist. HbA_{1c} kommt in kleinen Mengen auch in den Erythrozyten gesunder Menschen vor, tritt aber, in Abhängigkeit vom langfristigen Blutzuckerspiegel, vermehrt bei Diabetikern auf. HbA_{1c} eignet sich daher, entsprechend der mittleren Lebensdauer der Erythrozyten von 3 bis 4 Monaten, insbesondere zu einer rückwirkenden Beurteilung des Kohlenhydrathaushalts dieser Patienten und ergänzt als langfristiger Parameter neben dem kurzfristigen Parameter Blutglukosegehalt das Monitoring des Blutzuckerspiegels. Hierbei kommt der HbA_{1c}-Bestimmung eine große diagnostische Bedeutung zu.

Als Indikatorsubstanz können insbesondere fluoreszenzmarkierte Boronsäuren eingesetzt werden, als entsprechende Fängersubstanzen in der Extraktionsschicht spezielle Zucker und Zuckerderivate, insbesondere Diole.

Eine Ausführungsform eines erfindungsgemäßen Testträger zur HbA_{1c}-Bestimmung aus Vollblut besteht aus eine Testträger, auf welchem eine Extraktionsschicht aufgebracht ist, welche als spezifische Fängersubstanz beispielsweise cis-Diole, wie z.B. Polyzucker oder Polyalkohole, in immobilisierter Form enthält. Auf dem Testträger wird weiterhin eine Reagenzgemisch, welches mindestens ein Lysereagenz, beispielsweise Saponine, und ein bestimmte Menge der fluoreszenzmarkierten Boronsäure enthält, welche beispielsweise mit Aminomethylcoumarin oder Pyrentrisulfonat markiert ist, aufgebracht, bevorzugt direkt auf die Oberfläche der zuvor aufgebrachten Extraktionsschicht. Nach Auftrag der Flüssigkeitsprobe, in diesem Falle Vollblut, wird die Reagenzgemisch gelöst und die Nachweisreaktion im Reaktionsraum, welcher in diesem Fall dem Volumen der Flüssigkeitsprobe entspricht, wird gestartet. Hierbei binden die fluoreszenzmarkierten Boronsäuren mit ihrer Borongruppe kovalent an die Glukoseeinheiten des HbA_{1c}, welches in der Flüssigkeitsprobe vorliegt. Die Menge der an Hämoglobin gebundenen fluoreszenzmarkierten Boronsäure korreliert mit der Konzentration von HbA_{1c} in der Probe. Je mehr HbA_{1c} in der Probe vorliegt, desto mehr liegen die fluoreszenzmarkierten Boronsäure an Hämoglobin gebundenen vor und desto geringer ist die Konzentration frei vorliegender fluoreszenzmarkierter Boronsäure im Reaktionsraum. Da die frei vorliegenden fluoreszenzmarkierten Boronsäure als Indikatorsubstanzen dienen, zeigt dieses Beispiel, dass Indikatorsubstanzen nicht unbedingt im Rahmen der Nachweisreaktionen im Reaktionsraum gebildet werden müssen, sondern dass auch eine Umsetzung bereits vorhandener Indikatorsubstanzen zur Analytbestimmung eingesetzt werden kann. Die Extraktionsschicht besitzt eine Ausschlussgröße, welche das Eindringen von Hämoglobin in diese nicht erlaubt, da Hämoglobin den fluorimetrischen Nachweis der fluoreszenzmarkierten Boronsäuren verhindern würde. Daher können auch die an Hämoglobin gebundenen fluoreszenzmarkierten Boronsäuren nicht in die Nachweisschicht eindringen. Stattdessen werden die frei vorliegenden fluoreszenzmarkierten Boronsäuren als Indikatorsubstanzen eingesetzt, da diese aufgrund ihrer deutlich kleineren Molekülgröße in die Extraktionsschicht eindringen können und sich dort aufgrund ihrer spezifischen Eigenschaften in Wechselwirkung mit den speziellen Fängersubstanzen anreichern. Je mehr HbA_{1c} in der Probe vorhanden ist, desto weniger frei vorliegende fluoreszenzmarkierte Boronsäure kann in die Extraktionsschicht eindringen und desto geringer ist das Messsignal. Der Nachweis der fluoreszenzmarkierten Boronsäuren erfolgt in der Extraktionsschicht mit Hilfe fluoreszenzoptischer Verfahren.Die diagnostische Eignung eines solchen erfindungsgemäßen Testsystems kann noch erhöht werden, indem es als Multi-Parameter-Testsystem ausgebildet ist. So können beispielsweise mehrere verschiedene Analyten in einem gemeinsamen Testsystem bestimmt werden, von denen bekannt ist, dass ihre Konzentrationen oder ihr generelles Fehlen oder Vorhandensein bei Vorliegen eines bestimmten Krankheitsbildes jeweils in charakteristischer Weise verändert sind. Durch das gleichzeitige Messen mehrerer solcher zusammenhängender Parameter können zeitgleich und mit einem einzigen Arbeitsgang verschiedene Analyten bestimmt werden, was eine rasche diagnostische Aussage ermöglicht. Weiterhin können durch Berücksichtigung von spezifischen Kombinationen der einzelnen Analyseergebnisse oft diagnostische Aussagen getroffen werden, welche durch die Betrachtung nur eines Parameters nicht möglich sind. Insbesondere kann so die Spezifität und/oder Sensitivität des diagnostischen Verfahrens gesteigert werden. Im vorliegenden Fall kann insbesondere eine HbA_{1c}-Bestimmung mit der Bestimmung des Gesamthämoglobingehaltes in der Blutprobe kombiniert werden. Diese beiden Messwerte können anschließend in Beziehung gesetzt werden. Insbesondere kann der Anteil des HbA1c am Gesamthämoglobingehalt bestimmt werden, so dass aufgrund dieser gemeinsamen Betrachtungsweise exaktere diagnostische Aussagen möglich sind.

## Patentansprüche

1. Verfahren zur Bestimmung eines Analyten in einer Flüssigkeitsprobe mittels eines Testsystems bestehend aus mindestens zwei Kompartimenten, wobei in einem ersten Kompartiment die zur Bestimmung des Analyten notwendigen Nachweisreaktionen durchgeführt werden und in einem zweiten Kompartiment eine analytische Bestimmung einer an den Nachweisreaktionen beteiligten und vom Analyten verschiedenen Substanz, der Indikatorsubstanz, erfolgt, wobei die beiden Kompartimente in einer Weise voneinander abgegrenzt sind, welche den Übertritt der Indikatorsubstanz in das zweite Kompartiment ermöglicht und welche den Übertritt von weiteren Substanzen, welche die analytische Bestimmung der Indikatorsubstanz im zweiten Kompartiment stören können, zumindest teilweise verhindern und
im zweiten Kompartiment mindestens eine weitere Substanz in immobilisierter Form vorliegt, welche als Fängersubstanz eine selektive Anreicherung der Indikatorsubstanz im zweiten Kompartiment bewirkt,
**dadurch gekennzeichnet, dass**
a) der Analyt glycosyliertes Hämoglobin und die Flüssigkeitsprobe Vollblut oder ein davon abgeleitetes Produkt ist,
b) im Laufe der Nachweisreaktionen im ersten Kompartiment eine niedermolekulare fluoreszenzmarkierte Boronsäure als Indikatorsubstanz vorliegt, welche spezifisch an glycosyliertes Hämoglobin bindet und welche ein Molekulargewicht kleiner als 15.000 g/mol, vorzugsweise kleiner als 2.000 g/mol, besonders vorzugsweise kleiner als 1.500 g/mol besitzt, und
c) die nicht an glycosyliertes Hämoglobin gebundene Indikatorsubstanz durch spezifische Fängersubstanzen, insbesondere Kohlenhydrate und Diole, im zweiten Kompartiment unter Ausschluss störender Probenbestandteile, insbesondere Blutzellen und chromophorer Substanzen wie Hämoglobin, und unter Ausschluß der an glycosyliertes Hämoglobin gebundene Indikatorsubstanz angereichert wird und
d) der Nachweis der Indikatorsubstanz im zweiten Kompartiment mit optischen Methoden, insbesondere fluoreszenzoptischen Methoden, erfolgt.

2. Verfahren gemäß einem der vorhergehenden Ansprüche,
**dadurch gekennzeichnet, dass**
zur Bestimmung von glycosyliertem Hämoglobin chemische Nachweisreaktionen im ersten Kompartiment stattfinden, in welchen die Indikatorsubstanz in einer Weise umgesetzt wird, welche mit dem Vorliegen und insbesondere mit der Konzentration von glycosyliertem Hämoglobin im Vollblut oder einem davon abgeleitetem Produkt korreliert, insbesondere aufgrund stöchiometrischer Beziehungen.

3. Verfahren gemäß einem der vorhergehenden Ansprüche,
**dadurch gekennzeichnet, dass**
die Abgrenzung der beiden Kompartimente durch die Ausgestaltung des zweiten Kompartiments in Form einer selektiv permeablen Matrix, insbesondere eines Geles, eines Filmes, einer Membran oder einer Polymerschicht erfolgt.

4. Verfahren gemäß einem der vorhergehenden Ansprüche,
**dadurch gekennzeichnet, dass**
die selektive Anreicherung der Indikatorsubstanz in zweiten Kompartiment auf spezifischen hydrophilen/hydrophoben Wechselwirkungen zwischen der Indikatorsubstanz und der Fängersubstanz beruht.

5. Verfahren gemäß einem der vorhergehenden Ansprüche,
**dadurch gekennzeichnet, dass**
die Fängersubstanzen im zweiten Kompartiment cis-Diole, insbesondere Polyzucker oder Polyalkohole, sind.

6. Verfahren gemäß einem der vorhergehenden Ansprüche,
**dadurch gekennzeichnet, dass**
die Immobilisierung der Fängersubstanz durch Einschluss in eine Matrix erfolgt.

7. Verfahren gemäß einem der vorhergehenden Ansprüche,
**dadurch gekennzeichnet, dass**
die Matrix, welche die Immobilisierung der Fängersubstanz bewirkt, gleichzeitig die Abgrenzung der beiden Kompartimente bewirkt.

8. Testsystem zur Bestimmung von glycosyliertem Hämoglobin als Analyten in Vollblut oder einem davon abgeleitetem Produkt als Flüssigkeitsprobe, bestehend aus mindestens zwei Kompartimenten, wobei in einem ersten Kompartiment die zur Bestimmung von glycosyliertem Hämoglobin notwendigen Nachweisreaktionen durchgeführt werden und in einem zweiten Kompartiment eine analytische Bestimmung einer an den Nachweisreaktionen beteiligten und von glycosyliertem Hämoglobin verschiedenen Substanz, der Indikatorsubstanz, erfolgt, wobei
die beiden Kompartimente in einer Weise voneinander abgegrenzt sind, welche den Übertritt der Indikatorsubstanz in das zweite Kompartiment ermöglicht und welche den Übertritt von weiteren Substanzen, welche die analytische Bestimmung der Indikatorsubstanz im zweiten Kompartiment stören können, zumindest teilweise verhindern und
im zweiten Kompartiment mindestens eine weitere Substanz in immobilisierter Form vorliegt, welche als Fängersubstanz eine selektive Anreicherung der Indikatorsubstanz im zweiten Kompartiment bewirkt,
**dadurch gekennzeichnet, dass**
a) zur Durchführung der Nachweisreaktionen im ersten Kompartiment eine niedermolekulare fluoreszenzmarkierte Boronsäure als Indikatorsubstanz vorliegt, welche spezifisch an glycosyliertes Hämoglobin bindet und welche ein Molekulargewicht kleiner als 15.000 g/mol, vorzugsweise kleiner als 2.000 g/mol, besonders vorzugsweise kleiner als 1.500 g/mol besitzt, und
b) die nicht an glycosyliertes Hämoglobin gebundene Indikatorsubstanz durch spezifische Fängersubstanzen, insbesondere Kohlenhydrate und Diole, im zweiten Kompartiment unter Ausschluss störender Probenbestandteile, insbesondere Blutzellen und chromophorer Substanzen wie Hämoglobin, und unter Ausschluß der an glycosyliertes Hämoglobin gebundene Indikatorsubstanz angereichert wird und
c) der Nachweis der Indikatorsubstanz im zweiten Kompartiment mit optischen Methoden, insbesondere fluoreszenzoptischen Methoden, erfolgt.

## Claims

1. Method for determining an analyte in a liquid sample by means of a test system consisting of at least two compartments, wherein the detection reactions necessary for determining the analyte are carried out in a first compartment and an analytical determination of a substance, the indicator substance, that is involved in the detection reactions and is different from the analyte takes place in a second compartment, wherein the two compartments are delimited from one another in a manner which allows the indicator substance to pass into the second compartment and which at least partially prevents passage of other substances which can interfere with the analytical determination of the indicator substance in the second compartment and at least one other substance is present in an immobilized form in the second compartment which, as a capture substance, selectively enriches the indicator substance in the second compartment,
**characterized in that**
a) the analyte is glycosylated haemoglobin and the liquid sample is whole blood or a product derived therefrom,
b) a low-molecular-weight, fluorescently-labelled boronic acid which specifically binds to glycosylated haemoglobin and which has a molecular weight of less than 15,000 g/mol, preferably less than 2,000 g/mol and particularly preferably less than 1,500 g/mol is present as an indicator substance in the first compartment during the detection reactions, and
c) the indicator substance that has not bound to glycosylated haemoglobin is enriched by specific capture substances, in particular carbohydrates and diols, in the second compartment while excluding interfering sample components, in particular blood cells and chromophoric substances such as haemoglobin and while excluding the indicator substance bound to glycosylated haemoglobin and
d) the indicator substance is detected in the second compartment using optical methods, in particular fluorescent optical methods.

2. Method according to one of the previous claims,
**characterized in that**
chemical detection reactions take place in the first compartment in order to determine glycosylated haemoglobin in which the indicator substance is converted in a manner which correlates with the presence and in particular with the concentration of glycosylated haemoglobin in whole blood or in a product derived therefrom and in particular on the basis of stoichiometric relationships.

3. Method according to one of the previous claims,
**characterized in that**
the two compartments are delimited by designing the second compartment in the form of a selectively permeable matrix, in particular a gel, a film, a membrane or a polymer layer.

4. Method according to one of the previous claims,
**characterized in that**
the selective enrichment of the indicator substance in the second compartment is based on specific hydrophilic/hydrophobic interactions between the indicator substance and the capture substance.

5. Method according to one of the previous claims,
**characterized in that**
the capture substances in the second compartment are cis-diols, in particular polysugars or polyalcohols.

6. Method according to one of the previous claims,
**characterized in that**
the capture substance is immobilized by enclosure in a matrix.

7. Method according to one of the previous claims,
**characterized in that**
the matrix which immobilizes the capture substance at the same time delimits the two compartments.

8. Test system for determining glycosylated haemoglobin as an analyte in whole blood or a product derived therefrom as a liquid sample consisting of at least two compartments, wherein the detection reactions necessary for determining glycosylated haemoglobin are carried out in a first compartment and an analytical determination of a substance, the indicator substance, that is involved in the detection reactions and is different from glycosylated haemoglobin takes place in a second compartment, wherein
the two compartments are delimited from one another in a manner which allows the indicator substance to pass into the second compartment and which at least partially prevents passage of other substances which can interfere with the analytical determination of the indicator substance in the second compartment and
at least one other substance is present in an immobilized form in the second compartment which, as a capture substance, selectively enriches the indicator substance in the second compartment,
**characterized in that**
a) a low-molecular-weight, fluorescently-labelled boronic acid which specifically binds to glycosylated haemoglobin and which has a molecular weight of less than 15,000 g/mol, preferably less than 2,000 g/mol and particularly preferably less than 1,500 g/mol is present as an indicator substance in order to carry out the detection reactions in the first compartment, and
b) the indicator substance that has not bound to glycosylated haemoglobin is enriched by specific capture substances, in particular carbohydrates and diols, in the second compartment while excluding interfering sample components, in particular blood cells and chromophoric substances such as haemoglobin and while excluding the indicator substance bound to glycosylated haemoglobin and
c) the indicator substance is detected in the second compartment using optical methods, in particular fluorescent optical methods.

## Revendications

1. Procédé pour la détermination d'un analyte dans un échantillon liquide au moyen d'un système test, constitué par au moins deux compartiments, les réactions de détection nécessaires pour la détermination de l'analyte étant réalisées dans un premier compartiment et une détermination analytique d'une substance participant aux réactions de détection et différente de l'analyte, la substance indicatrice, ayant lieu dans un deuxième compartiment, les deux compartiments étant délimités l'un de l'autre d'une manière qui permet le passage de la substance indicatrice dans le deuxième compartiment et qui empêche au moins partiellement le passage d'autres substances, qui peuvent perturber la détermination analytique de la substance indicatrice dans le deuxième compartiment et au moins une autre substance se trouvant sous forme immobilisée dans le deuxième compartiment, provoquant, en tant que substance piège, un enrichissement sélectif de la substance indicatrice dans le deuxième compartiment, **caractérisé en ce que**
a) l'analyte est l'hémoglobine glycosylée et l'échantillon liquide est du sang complet ou un produit dérivé de celui-ci,
b) un acide boronique marqué par une fluorescence, de bas poids moléculaire, se trouve au cours des réactions de détection dans le premier compartiment en tant que substance indicatrice, qui se lie spécifiquement à l'hémoglobine glycosylée et qui présente un poids moléculaire inférieur à 15 000 g/mole, de préférence inférieur à 2000 g/mole, en particulier de préférence inférieur à 1500 g/mole, et
c) la substance indicatrice non liée à l'hémoglobine glycosylée est enrichie par des substances piège spécifiques, en particulier des hydrates de carbone et des diols, dans le deuxième compartiment, en excluant des constituants perturbants de l'échantillon, en particulier des cellules sanguines et des substances chromophores, telles que l'hémoglobine, et en excluant la substance indicatrice liée à l'hémoglobine glycosylée et
d) la détection de la substance indicatrice dans le deuxième compartiment a lieu par des procédés optiques, en particulier des procédés optiques de fluorescence.

2. Procédé selon revendication précédente, **caractérisé en ce que** pour la détermination de l'hémoglobine glycosylée, des réactions de détection chimiques ont lieu dans le premier compartiment, par lesquelles la substance indicatrice est transformée d'une manière qui corrèle avec la présence et en particulier avec la concentration de l'hémoglobine glycosylée dans le sang complet ou un produit dérivé de celui-ci, en particulier sur base de rapports stoechiométriques.

3. Procédé selon l'une quelconque des revendications précédentes, **caractérisé en ce que** la délimitation des deux compartiments a lieu par la réalisation du deuxième compartiment sous forme d'une matrice à perméabilité sélective, en particulier d'un gel, d'un film, d'une membrane ou d'une couche polymère.

4. Procédé selon l'une quelconque des revendications précédentes, **caractérisé en ce que** l'enrichissement sélectif de la substance indicatrice dans le deuxième compartiment repose sur des interactions hydrophiles/hydrophobes spécifiques entre la substance indicatrice et la substance piège.

5. Procédé selon l'une quelconque des revendications précédentes, **caractérisé en ce que** les substances piège dans le deuxième compartiment sont des diols cis, en particulier des polysucres ou des polyalcools.

6. Procédé selon l'une quelconque des revendications précédentes, **caractérisé en ce que** l'immobilisation de la substance piège a lieu par incorporation dans une matrice.

7. Procédé selon l'une quelconque des revendications précédentes, **caractérisé en ce que** la matrice qui provoque l'immobilisation de la substance piège, provoque simultanément la délimitation des deux compartiments.

8. Système test pour la détermination de l'hémoglobine glycosylée en tant qu'analyte dans du sang complet ou un produit dérivé de celui-ci comme échantillon liquide, constitué par au moins deux compartiments, les réactions de détection nécessaires pour la détermination de l'hémoglobine glycosylée étant réalisées dans un premier compartiment et une détermination analytique d'une substance participant aux réactions de détection et différente de l'hémoglobine glycosylée, la substance indicatrice, ayant lieu dans un deuxième compartiment, les deux compartiments étant délimités l'un de l'autre d'une manière qui permet le passage de la substance indicatrice dans le deuxième compartiment et qui empêche au moins partiellement le passage d'autres substances, qui peuvent perturber la détermination analytique de la substance indicatrice dans le deuxième compartiment et au moins une autre substance se trouvant sous forme immobilisée dans le deuxième compartiment, provoquant, en tant que substance piège, un enrichissement sélectif de la substance indicatrice dans le deuxième compartiment, **caractérisé en ce que**
a) un acide boronique marqué par une fluorescence, de bas poids moléculaire, se trouve dans le premier compartiment pour la réalisation des réactions de détection, en tant que substance indicatrice, qui se lie spécifiquement à l'hémoglobine glycosylée et qui présente un poids moléculaire inférieur à 15 000 g/mole, de préférence inférieur à 2000 g/mole, en particulier de préférence inférieur à 1500 g/mole, et
b) la substance indicatrice non liée à l'hémoglobine glycosylée est enrichie par des substances piège spécifiques, en particulier des hydrates de carbone et des diols, dans le deuxième compartiment, en excluant des constituants perturbants de l'échantillon, en particulier des cellules sanguines et des substances chromophores, telles que l'hémoglobine, et en excluant la substance indicatrice liée à l'hémoglobine glycosylée et
c) la détection de la substance indicatrice dans le deuxième compartiment a lieu par des procédés optiques, en particulier des procédés optiques de fluorescence.
